(19) Europäisches Patentamt
European Patent Office
Office européen des brevets

(11) **EP 4 410 197 A1**

(12) **EUROPEAN PATENT APPLICATION**
published in accordance with Art. 153(4) EPC

(43) Date of publication:
**07.08.2024 Bulletin 2024/32**

(21) Application number: **22901480.8**

(22) Date of filing: **02.06.2022**

(51) International Patent Classification (IPC):
**A61B 5/145** (2006.01) **A61B 5/155** (2006.01)
**A61B 5/00** (2006.01) **G16H 50/20** (2018.01)

(52) Cooperative Patent Classification (CPC):
**A61B 5/00; A61B 5/145; A61B 5/155; G16H 50/20**

(86) International application number:
**PCT/KR2022/007876**

(87) International publication number:
**WO 2023/101119 (08.06.2023 Gazette 2023/23)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **03.12.2021 KR 20210171649**

(71) Applicant: **i-Sens, Inc.**
**Seoul 06646 (KR)**

(72) Inventors:
• **LEE, David**
  **Seoul 06646 (KR)**
• **KANG, Young Jea**
  **Seoul 06646 (KR)**
• **NAM, Hak Hyun**
  **Seoul 06646 (KR)**

(74) Representative: **BCKIP Part mbB**
**Siegfriedstraße 8**
**80803 München (DE)**

(54) **BIOMETRIC VALUE PREDICTION METHOD**

(57) The present invention relates to a method for predicting a biometric value in a blood glucose measurement system and, more particularly, to a biometric value prediction method capable of predicting a future biometric value of a user by generating a predictive model through a communication terminal having a small memory and amount of calculations, such as a smartphone that the user always carries to manage a biometric value, and applying the biometric value of the user to the generated predictive model, and capable of predicting a future biometric value of the user without requiring biometric information of other nearby users and without access to a server, by generating a predictive model personalized for the user on the basis of biometric history information of the user.

Fig. 5

EP 4 410 197 A1

**Description**

**TECHNICAL FIELD**

The present disclosure relates to a method for predicting a biometric value in a blood glucose measurement system, and more specifically, to a biometric value prediction method for predicting a future biometric value of a user by generating a prediction model through a communication terminal having a small memory and computational amount, such as a smartphone that the user always possesses to manage the biometric value, applying the biometric information of the user to the generated prediction model, and creating a personalized prediction model to the user based on the biometric information, thereby predicting the future biometric value of the user without requiring biometric information from other nearby users and without access to a server.

**BACKGROUND**

Diabetes is a chronic medical condition that is common in modern people, and in the Republic of Korea, there are 2 million diabetes patients, about 5% of the total population.

Diabetes occurs when the absolute level of the sugar level in blood is high due to the absolute deficiency or relative insufficiency of insulin, produced by the pancreas, caused by various reasons such as obesity, stress, poor eating habits, and inherited hereditary factors and imbalance regarding glucose in the blood.

The blood usually contains a certain concentration of glucose, and tissue cells gain energy from the glucose.

However, when the glucose is increased excessively more than needed, the glucose cannot be properly stored in the liver, muscle, or adipose tissue and is accumulated in the blood, because of this, patients with diabetes maintain a much higher blood glucose level than normal people, and as excessive blood glucose passes through the tissues and is discharged into the urine, it results in deficiency of glucose, which is absolutely necessary for all tissues of the body, thereby causing abnormalities in respective body tissues.

Diabetes is characterized by substantial absence of subjective symptoms at the beginning of the condition, when diabetes progresses, diabetes-specific symptoms such as overdrink, overeat, polyuria, weight loss, weariness, skin itchiness, and lower ability of naturally healing on injury on hands and feet are shown, and further progression of diabetes leads to complications such as visual disturbances, hypertension, kidney disease, paralysis, periodontal disease, muscle spasms and neuralgia, as well as gangrene.

In order to diagnose diabetes beforehand and manage to prevent the progression of diabetes into complications associated therewith, systematic blood glucose measurement and treatment should be performed.

Diabetes needs to constantly measure blood glucose for management, so the demand for devices related to blood glucose measurement is steadily increasing. It has been confirmed through various studies that, when diabetic patients strictly control the management of blood glucose, the incidence of complications of diabetes is significantly reduced. Accordingly, it is very important for diabetic patients to measure blood glucose regularly for blood glucose management.

The finger prick method is generally used to manage blood glucose level in diabetic patients, and although such a blood collecting glucose measurement device helps diabetics patients manage blood glucose level, it is difficult to accurately identify frequently changing blood glucose levels because only the results at the time of measurement appear. In addition, the finger prick method requires blood collection to measure blood glucose levels frequently throughout the day, which poses a significant burden on diabetic patients.

Diabetics patients generally experience hyperglycemia and hypoglycemia, and an emergency may occur in the hypoglycemic conditions. Hypoglycemia occurs when sugar content is not kept for a long time, and the patients may become unconscious or die in the worst case. Accordingly, rapid discovery of the hypoglycemic condition is critically important for diabetics. The blood-gathering-type biometric information measuring device intermittently measuring glucose has limited ability to accurately measure blood glucose levels.

Recently, to overcome such a drawback of the blood-collecting-type biometric information measuring device, continuous glucose monitoring systems (CGMSs) inserted into the human body to measure a blood glucose level every few minutes have been developed, and therefore easily perform the management of diabetics and responses to an emergency situation.

The continuous blood glucose monitoring system includes a body attachable unit that is inserted into the human body and measures blood glucose level using body fluids such as blood, and a communication terminal for communicating with the body attachable unit and displaying a blood glucose level measured in the body attachable unit.

The body attachable unit consists of a sensor that is partially inserted into the body for a certain period, for example less than 3 months (7 weeks, 15 days, 1 month, etc.) and generates biosignals representing the blood glucose level of a user from body fluids and a transmitter that transmits biosignals received from a sensor to a communication terminal in real time, periodically, or upon request from the communication terminal.

The body attachable unit generates biosignals and transmits them to the communication terminal, and a blood glucose management application is installed in the communication terminal to receive biosignals from the body attachable unit and output biosignals so that a user may check them after procedures such as preprocess like noise removal from the received biosignal, unit calibration of the biosignal from the received current value to the blood glucose level, and calibration using the reference blood glucose level.

In this way, the continuous blood glucose measurement system not only measures the biosignals of a user in real time and outputs blood glucose level of a user, but also predict the future blood glucose level of a user based on the user's blood glucose level history, activity history, meal history, and medication administration history such as insulin.

Prediction of the future blood glucose level can be done in various ways, and typically, a prediction model is created based on the blood glucose level history of a user and the blood glucose level history of other users around the user, and by applying the current blood glucose level of the user to the generated prediction model, the future blood glucose level of the user can be predicted.

This technology for predicting the future blood glucose level has the effect of predicting the possibility of a user falling into hypoglycemia or hyperglycemia in advance, allowing a user to respond in advance before reaching the actual hypoglycemia or hyperglycemia situation.

However, since the prediction model conventionally used to predict future blood glucose levels is created using extensive blood glucose history information, meal history information, activity history, etc. of not only a user but also other surrounding users, so there is a problem that a very large memory space is required to store the information needed to create the prediction model, and a large amount of calculation is required to predict future blood glucose levels by applying the biosignals of a user to the prediction model.

Therefore, the creation and modification of these prediction models and the prediction of future blood glucose levels using the prediction model must be mainly done through a separate server, and there is a problem in that the communication terminal must always communicate with the server to predict future blood glucose levels.

## DETAILED DESCRIPTION OF DISCLOSURE

### Technical Problem

As the present disclosure is intended to solve the problems of the conventional method for predicting future blood glucose levels mentioned above, the purpose of the present disclosure is to provide a biometric value prediction method that can predict the future blood glucose level of a user with a small memory and computational amount through a communication terminal such as a smartphone which a user always carries to manage blood glucose levels.

Another purpose of the present disclosure is to create a prediction model which is personalized to a user based on the biometric history information and provide a method for predicting future biometric values without access to a server.

Another purpose of the present disclosure is to provide a biometric value prediction method that determine the expression characteristics of the prediction error by calculating the prediction error from the predicted biometric values and actual biometric values and can accurately predict the future biometric values of a user by relearning or regenerating the prediction model according to the expression characteristics of the prediction error.

Another purpose of the present disclosure is to create a personalized prediction model for a user using a first feature value extracted from preprocessed biometric information and a second feature value extracted from the time-calibrated biometric information and unit-calibrated biometric information, and can provide a method for accurately predicting the biometric values of a user through the generated prediction model.

### Solution to Problem

To accomplish the above-described purposes, according to an embodiment of the present disclosure, a method of predicting a biometric value of a user using biometric information measured from a sensor may comprise: extracting a first feature value from the measured biometric information of the user; calibrating the measured biometric information of the user and extracting a second feature value from the calibrated biometric information; generating a feature vector value by reducing and combining the first feature value and the second feature value; and predicting the biometric value of the user by applying the generated feature vector value to a prediction model.

Here, the sensor is a sensor partially inserted into body of the user for a certain period of time and continuously measuring the biometric information of the user.

Preferably, the method of predicting the biometric value may further include pre-processing the measured biometric information by removing noise from the measured biometric information, wherein the first feature value and the second feature value are extracted from the pre-processed biometric information.

Here, the first feature value may be directly extracted from the pre-processed biometric information, and the second feature value may be extracted from the calibrated biometric information generated by calibrating the pre-processed biometric information with respect to time delay and unit discrepancy.

Here, the unit discrepancy may be calibrated based on the pre-processed biometric information or a reference biometric value.

According to an embodiment of the present disclosure, the unit discrepancy may be calibrated by assigning a weight when the pre-processed biometric information increases or decreases.

According to another embodiment of the present disclosure, the unit discrepancy may be calibrated by a weight assigned according to difference between the biometric value determined from the measured biometric information and the reference biometric value.

Preferably, the method of predicting the biometric value may further comprise: calculating a prediction error from a difference between a predicted biometric value at a first prediction time and a biometric value actually measured at the first prediction time, and determining whether to re-learn the prediction model based on the prediction error.

Here, if the prediction error is greater than a threshold or a threshold ratio, it may be determined that the prediction model is to be re-learned.

In the method of predicting the biometric value according to an embodiment of the present disclosure, the re-learning of the prediction model or re-generating of the prediction model may use a subsequent data set generated from biometric information of the user measured up to current time except a previous data set which was used to create the prediction model.

Preferably, the method of predicting the biometric value according to an embodiment of the present disclosure may further comprise determining whether to re-generate the prediction model based on expression characteristics of the prediction error during a unit time.

Here, the expression characteristics may be at least one of a number of consecutive times of excess of the prediction error over the threshold or the threshold ratio during the unit time and a total number of times of excess of the prediction error over the threshold or the threshold ratio during the unit time.

## Advantageous Effects of Invention

The method for predicting biometric values according to the present disclosure has the following effects.

First, the method for predicting biometric values according to the present disclosure can predict the future biometric values of a user by generating a prediction model through a communication terminal with a small memory and computational amount, such as a smartphone which a user always carries to manage the biometric values, and by applying the biometric information of a user to the generated prediction model.

Second, the biometric value prediction method according to the present disclosure generates a prediction model which is personalized to a user based on the biometric history information of a user, so the future biometric values of a user can be predicted without requiring biometric information from other nearby users and without access to a server.

Third, the biometric value prediction method according to the present disclosure can accurately predict the future biometric values of a user by calculating the prediction error from the predicted biometric value and the actual biometric value, determining the expression characteristics of the prediction error, and relearning or regenerating the prediction model according to the characteristics of the prediction error.

Fourth, the method for predicting biometric values according to the present disclosure can accurately predict the biometric values of a user through the generated prediction model by generating a personalized prediction model for a user using a first feature value extracted from preprocessed biometric information and a second feature value extracted from the time-calibrated biometric information and unit-calibrated biometric information.

## BRIEF DESCRIPTION OF THE DRAWINGS

FIG. 1 is a schematic diagram showing a blood glucose measurement system according to an embodiment of the present disclosure.

FIG. 2 is a functional block diagram for explaining a biometric value prediction device according to the present disclosure.

FIG. 3 is a functional block diagram for explaining an example of a feature value generator unit according to the present disclosure.

FIG. 4 is a functional block diagram for explaining an example of a learning unit according to the present disclosure.

FIG. 5 is a flowchart to explain the method for predicting biometric values according to the present disclosure.

FIG. 6 is a flowchart to explain an example of the step of relearning a prediction model in the present disclosure.

FIG. 7 is a flowchart to explain an example of the step of regenerating a prediction model in the present disclosure.

FIG. 8 is a diagram for explaining an example of time delay calibration.

FIG. 9 is a diagram for explaining an example of a method for calibrating unit discrepancy.

FIG. 10 is a diagram for explaining another example of a method for calibrating unit discrepancy.

FIGS. 11 and 12 are diagrams for explaining an example of determining whether to relearn a prediction model.

FIG. 13 is a diagram for explaining an example of regenerating a prediction model.

## DESCRIPTION OF EMBODIMENTS OF DISCLOSURE

It should be noted that the technical terms used in the present disclosure are only used to describe specific embodiments and are not intended to limit the present disclosure. In addition, the technical terms used in the present disclosure, unless specifically defined in a different sense in the present disclosure, should be interpreted as meanings generally understood by those skilled in the art in the technical field to which the present disclosure belongs, and should not be interpreted as an excessively comprehensive sense or an excessively reduced sense. In addition, if the technical term used in the present disclosure is an incorrect technical term that does not accurately express the idea of the present disclosure, it should be replaced with a technical term that can be correctly understood by a person skilled in the art.

Additionally, as used in the present disclosure, singular expressions include plural expressions unless the context clearly dictates otherwise. In the present disclosure, terms such as "consists of" or "comprises" should not be construed as necessarily including all of the various components or steps described in the disclosure and should be interpreted that some of the components or steps may not be included, or additional components or steps may be included.

In addition, it should be noted that the attached drawings are only intended to facilitate easy understanding of the concepts of the present disclosure, and should not be construed as limiting the concepts of the present disclosure by the attached drawings.

Hereinafter, the method for predicting biometric values according to the present disclosure will be described in more detail with reference to the attached drawings.

Hereinafter, a continuous blood glucose measurement system will be described that continuously measures biometric information indicating blood glucose level by a body attachable unit attached to the body of a user for a certain period of time and transmits the measured biometric information to a communication terminal, but depending on the field to which the present disclosure is applied, the body attachable unit can measure various types of biometric information and transmit the measured biometric information to a communication terminal, which falls within the scope of the present disclosure.

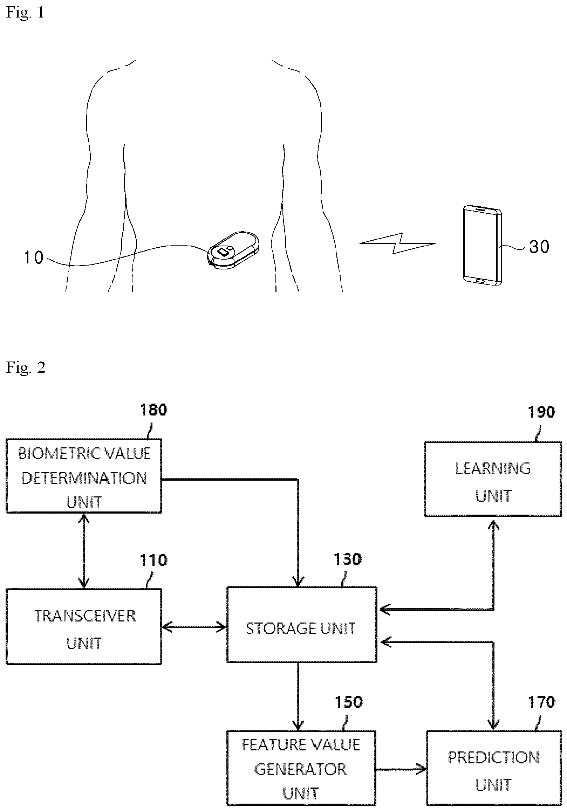

FIG. 1 is a schematic diagram showing a blood glucose measurement system according to an embodiment of the present disclosure.

Referring to FIG. 1, a blood glucose measurement system (1) according to an embodiment of the present disclosure includes a body attachable unit (10) and a communication terminal (30).

The body attachable unit (10) is attached to a body, and when the body attachable unit (10) is attached to the body, one end of the sensor of the body attachable unit (10) is inserted into a skin and continuously measures biometric information indicating a blood glucose level of a user using body fluids, etc.

The communication terminal (30) is a terminal that can receive biometric information from the body attachable unit (10) and display the received biometric information to a user, and for example, a mobile terminal that can communicate with the body attachable unit (10), such as a smartphone, tablet PC, or laptop, can be used. Of course, the communication terminal (30) is not limited thereto, and may be any type of terminal may be used as long as it includes a communication function and a program or application may be installed.

The body attachable unit (10) transmits the measured biometric information to the communication terminal (30) at the request of the communication terminal (30) or at a set time, and for data communication between the body attachable unit (10) and the communication terminal (30), the body attachable unit (10) and the communication terminal (30) may be connected to each other through a wired communication method such as a USB cable or a wireless communication method such as infrared communication, NFC communication, or Bluetooth.

The communication terminal (30) predicts the future biometric values of a user based on the received biometric information and provides the predicted biometric values to a user. Preferably, the communication terminal (30) may provide a hyperglycemia or hypoglycemia alarm to the user based on the predicted biometric values, or may provide a necessary prescription to a user along with the hypoglycemia or hypoglycemia alarm.

Here, the communication terminal (30) can store the received biometric information for a certain period of time, and the communication terminal (30) can create a prediction model using the stored biometric information.

Additionally, the communication terminal (30) monitors and compares the predicted biometric values for a certain time in the future with the actual biometric values actually measured after a certain time has elapsed, and may relearn or regenerate a prediction model generated based on the prediction error between the predicted biometric value and the actual biometric value or based on the expression characteristics of the prediction error.

FIG. 2 is a functional block diagram for explaining a biometric value prediction device according to the present disclosure.

The biometric value prediction device according to the present disclosure can be implemented in the communication terminal, and looking more closely with reference to FIG. 2, a transceiver unit (110) receives the biometric information of a user from a body attachable unit, and a storage unit (130) stores the received biometric information. Here, the storage unit (130) can map the received biometric information to the reception time and store it.

A biometric value determination unit (180) determines the biometric value of a user from the received biometric information and outputs the determined biometric value to the user or stores outputs the determined biometric value in the storage unit (130) by mapping it to the reception time.

Here, various types of biometric information can be measured through a sensor which is inserted into the body of a user for a certain period of time. Hereinafter, an example of biometric information may be biometric information representing the blood glucose information of a user, and a biometric value may be a blood glucose level determined from the biometric information.

The storage unit (130) may store biometric values determined from the biometric information along with biometric information received from the body attachable unit, and a feature value generator unit (150) uses biometric information to generate a feature value vector used to determine predicted biometric values at a future time point.

A prediction unit (170) uses the prediction model stored in the storage unit (130) to determine the predicted biometric value after a certain period of time based on the current time, and the prediction unit (170) determines the predicted biometric value by applying the generated feature value vector to the prediction model. Here, the predicted biometric value is an expected biometric value that a user will have after a certain period of time based on the current time, and using the predicted biometric values, the biometric value after a certain period of time can be predicted in advance, and an alarm or necessary prescription can be provided to a user based on the predicted biometric values.

For example, if the predicted biometric value after 3 hours from the current time is determined to be 60 mg/dL or less, a low blood glucose alarm may be provided to a user or a prescription to consume food may be provided.

Meanwhile, the storage unit (130) stores the predicted biometric values which are determined by the prediction unit (170) and the actual biometric values which are determined by the biometric value determination unit (180), and a learning unit (190) monitors and compares the actual biometric values determined after a certain time and the predicted biometric values predicted before a certain time.

The learning unit (190) may relearn the prediction model stored in the storage unit (130) based on the size or ratio of prediction errors between the predicted biometric values and the actual biometric values, or may learn the prediction model again from the beginning based on the expression characteristics of the prediction error, regenerate a new prediction model, and update the prediction model stored in the storage unit (130) with the generated new prediction model.

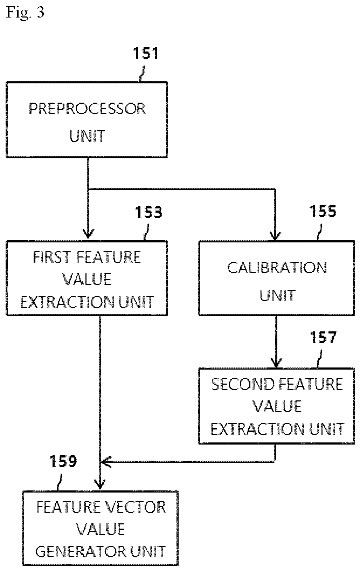

FIG. 3 is a functional block diagram for explaining an example of a feature value generator unit according to the present disclosure.

Looking more specifically with reference to FIG. 3, a preprocessor unit (151) removes or reduces noise from biometric information received by the transmitter and receiver.

Biometric information measured by or received from a body attachable unit may contain noise. For example, since the sensors of the body attachable unit are partially inserted into the human body, the sensor may move whenever a person moves, and as these sensors move, noise may be included in the biometric information data measured by the body attachable unit.

Alternatively, when biometric information is transmitted from a body attachable unit to a communication terminal, it may be affected by surrounding electromagnetic waves, etc., and as a result, noise may be included in the biometric information received from the communication terminal.

The preprocessor unit (151) performs a filtering process for outlier processing on the received biometric information or performs a low-pass filtering process on the biometric information for which outlier processing has been completed.

A first feature value extraction unit (153) extracts the first feature value from the preprocessed biometric information, and a second feature value extraction unit (157) extracts the second feature value from the biometric information calibrated by a calibration unit (155). The preprocessed biometric information received from the body attachable unit is current value information that changes differently depending on the biometric values of a user, and the calibrated biometric information refers to biometric information which is calibrated for time delay or unit inconsistency of biometric information preprocessed in the calibration unit (155).

Preferably, the calibration unit (155) calibrates the preprocessed biometric information and provides the calibrated biometric information to the second feature value extraction unit (157), and the calibration unit calibrates the time delay of the preprocessed biometric information or calibrates the biometric information of the current value into a unit of biometric value, for example, a unit of blood glucose level.

A feature vector value generator unit (159) generates a feature vector value by simply combining the first feature value and the second feature value or by reducing and combining the first feature value and the second feature value using a method such as resource reduction.

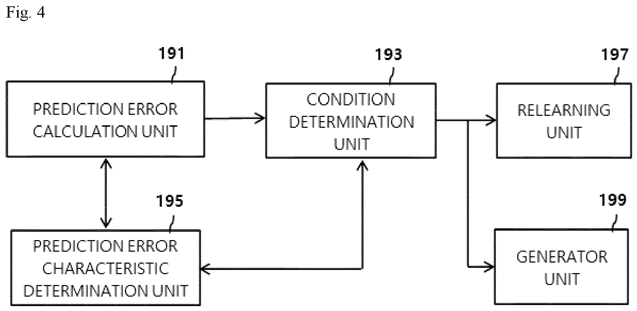

FIG. 4 is a functional block diagram for explaining an example of a learning unit according to the present disclosure.

Looking more specifically with reference to FIG. 4, a prediction error calculation unit (191) monitors and compares the actual biometric values determined after a certain period of time and the predicted biometric values predicted before a certain time, thereby calculating the prediction error between the actual biometric values and the predicted biometric values. For example, the predicted biometric value after t time has elapsed based on the current time is determined from the feature vector value, and the prediction error between the actual biometric value determined using biometric information after the actual t time and predicted biometric value has elapsed is calculated.

A prediction error characteristic determination unit (195) determines the expression characteristics of the prediction error, such as the number of times the prediction error exceeds the threshold or a threshold ratio during a unit time, or the number of times the prediction error continuously exceeds the threshold or the threshold ratio during the unit time.

A condition determination unit (193) determines the re-learning condition based on whether the prediction error exceeds a set threshold or whether the prediction error ratio compared to the actual biometric value or the predicted biometric value exceeds a set threshold ratio, or determines whether the regeneration conditions of the prediction model are satisfied based on the expression characteristics of the prediction error.

If the relearning condition is satisfied, a relearning unit (197) relearns the prediction model stored in the storage unit and updates the prediction model with the relearned prediction model, and if the regeneration condition is satisfied, a generator unit (199) creates a new prediction model stored in the storage unit and updates the prediction model with the newly created prediction model.

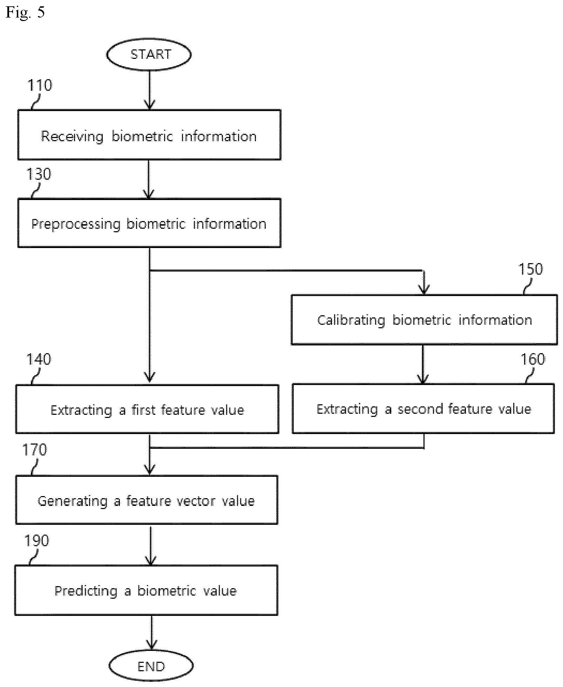

FIG. 5 is a flowchart to explain the method for predicting biometric values according to the present disclosure.

Looking more specifically with reference to FIG. 5, the biometric information of a user is received (S110). Here, the biometric information of a user can be received from a body attachable unit that is attached to the body of a user for a certain period of time and continuously measures the biometric information of a user.

The communication terminal preprocesses the received biometric information (S130). Looking at the preprocessing process in more detail, for outlier processing filtering, data outside a predetermined range among the received biometric information is found and the corresponding biometric information is processed. At this time, if it is determined that the biometric information has outliers, the biometric information can be removed and then processed. However, it is not limited to this, and biometric information with outliers can be calibrated and used as needed. Low-pass filtering can be performed on biometric information in which outliers are processed. Low-pass filtering removes components corresponding to high-band and leaves only biometric information corresponding to low-band. Using the low-pass filtered biometric information, the average of the low-pass filtered biometric information can be calculated and processed and the trimmed average can be used. Pretreatment is possible in various ways depending on the field to which the present disclosure is applied, and this falls within the scope of the present disclosure.

The first feature value is extracted from the preprocessed biometric information (S140). The preprocessed biometric information may be a biometric value of a user measured by a body attachable unit, for example, a current value indicating a blood glucose level. Here, the first feature value is a feature value such as difference, slope, deviation, average, effective value, and sharpness of biometric information extracted from statistical techniques, or a feature value extracted by frequency domain analysis of biometric information such as Fourier transform, wavelet transform, etc.

Meanwhile, the preprocessed biometric information is calibrated to generate calibrated biometric information (S150), and the second feature value is extracted from the calibrated biometric information (S150). Here, time delay or unit discrepancy, etc. included in the pre-processed biometric information can be calibrated before extracting the second feature value.

There is a time delay between the time when the biometric information of a user is actually measured by the body attachable unit and the time when the biometric information of a user is received by the communication terminal, and this time delay may occur depending on the physical structure until the sensor of the body attachable unit measures from the bodily fluids of a user and generates biometric information or may occur due to the computational time required to generate biometric information.

In addition, since the preprocessed biometric information is the current value information representing the biometric value of a user, not the biometric value itself, unit discrepancy must be calibrated from the current value to the actual biometric value of a user. Unit discrepancies can be calibrated using a reference biometric value measured by a blood collecting type biometric value meter using a separate sensor strip (blood test strip) and collected blood. For example, if the current value of the biometric information is 10nA and the reference biometric value measured at this time is 100mg/dL, the calibration slope (A) is set to 10 and the current value of the biometric information is then multiplied by the slope to determine the biometric value of a user.

Depending on the field to which the present disclosure is applied, the unit discrepancy can be calibrated by assigning weights depending on whether the biometric value is increasing or decreasing or the difference between the measured biometric value and the reference biometric value.

7

A second feature value is extracted using the calibrated biometric information (S 160). Here, the second feature value is a feature value such as difference, slope, deviation, average, effective value, and sharpness of the calibrated biometric information extracted from statistical techniques or a feature value extracted by frequency domain analysis of calibrated biometric information such as Fourier transform, wavelet transform, etc.

A feature vector value is generated from the first feature value and the second feature value by simply combining the first feature value and the second feature value or reducing resources (S170).

The generated feature vector value is applied to the prediction model and generates a predicted biometric value after a certain period of time (S190).

The prediction model is used to generate predicted biometric values after a certain time using a feature vector value, and for example, if the number of components that make up the feature vector value (x) generated at certain intervals is 10 and the label (y) for each feature vector value is the predicted biometric value after a certain time, the input (x) and label (y) of each feature vector value are expressed as Equation (1) below.

[Equation 1]

$$x = \begin{bmatrix} x_{1.1} & \cdots\cdots & x_{1.10} \\ \vdots & \vdots & \vdots \\ x_{n.1} & \cdots\cdots & x_{n.n} \end{bmatrix} \qquad y = \begin{bmatrix} y_1 & \cdots\cdots & y_n \end{bmatrix}$$

Based on this, if the feature vector value $x = [\, x_1 \,\cdots\cdots\, x_{10}]$ which is generated from feature value 1 and feature value 2 is entered into the prediction model, the predicted biometric value y' after a certain time is output.

Here, the prediction model uses the feature value vector of a user as training data and can be created using machine learning such as SVM (Support Vector Machine), GMM (Gaussian Mixture Model), deep learning such as CNN (Convolution Neural Network), reinforcement learning such as model-free RL and model-based RL, and deep reinforcement learning such as DQN (Deep Q Network). The prediction model created in the present disclosure can be relearned using additional or new training data. Various known techniques can be used to create a prediction model, and detailed descriptions thereof will be omitted.

The first feature value and the second feature value have many features. When generating a prediction model with such data, not only will the learning speed be slow, but the performance will also likely be poor because the dimension of the data is large. To this end, a prediction model can be created by simply combining the first feature value and the second feature value or by selecting or reducing features from them, and projection and manifold learning, which are methods for reducing the dimensionality of data, and principal component analysis (PCA), which is a representative dimensionality reduction algorithm, can be used, or feature selection algorithms such as Lasso can be used. Various known techniques can be used to generate feature vector values from the first feature value and the second feature value, and detailed description thereof will be omitted.

In the present disclosure, by creating a prediction model using both the first feature value and the second feature value generated from the biometric information of a user, it is possible to create a prediction model that is personalized to the user and can accurately predict the biometric value of a user even without using data from other nearby users, and has the effect of accurately predicting the biometric value of a user by applying the feature vector value generated from the first feature value and the second feature value to the prediction model.

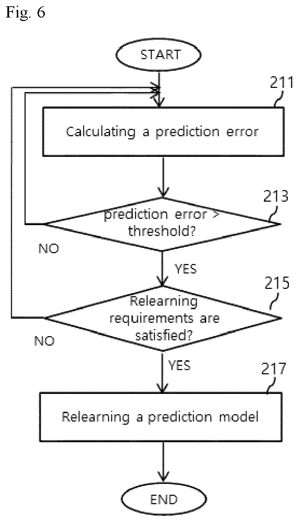

FIG. 6 is a flowchart to explain an example of the step of relearning a prediction model in the present disclosure.

Looking more specifically with reference to FIG. 6, the prediction error is calculated from the difference between the predicted biometric value of the first prediction time created using the feature vector value and the actual biometric value measured using the actual biometric value of the first prediction time (S211).

The prediction error is compared with a preset threshold or threshold ratio to determine whether the prediction error exceeds the threshold or threshold ratio (S213).

If the prediction error exceeds the threshold or threshold ratio, it is determined whether the relearning requirements are satisfied (S215). Here, the relearning requirement may be determined to be satisfied when the prediction error exceeds the threshold or the threshold rate, may be determined to be satisfied when the prediction error exceeds the threshold or threshold ratio and the average of the prediction errors for the preset first time period exceeds the average of thresholds, or may be determined to be satisfied when the prediction error exceeds the threshold or threshold ratio and then continuously exceeds the threshold or threshold ratio for the second time period.

If the relearning requirements are met, the prediction model is relearned, and the previously used prediction model is updated with the relearned prediction model (S217). Here, relearning the prediction model is characterized by using a subsequent data set generated from the biometric information measured up to the current time, in addition to the previous data set used to create the prediction model stored in the storage unit.

Here, the data set is characterized as a feature vector value generated from first feature values and second feature values.

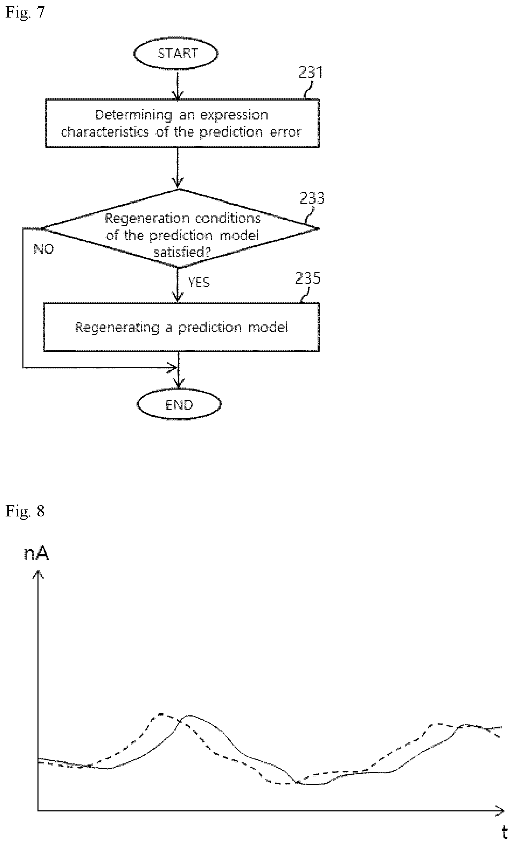

FIG. 7 is a flowchart to explain an example of the step of regenerating a prediction model in the present disclosure.

Looking in more detail with reference to FIG. 7, the expression characteristics of the prediction error are determined (S231). Here, the expression characteristics of the prediction error are the average of the prediction errors during a unit time, the number of times the prediction error exceeds the threshold or the threshold ratio during the unit time, the number of consecutive times the prediction error exceeds the threshold or the threshold ratio during the unit time, the ratio of time when the prediction error exceeds a threshold or threshold ratio relative to the unit time, etc.

Based on the expression characteristics of the prediction error, it is determined whether the regeneration conditions of the prediction model are satisfied (S233). Here, the condition for regenerating the prediction model may be that the average of the prediction errors during a unit time exceeds the first threshold average value, the number of times the prediction error exceeds the threshold value or threshold rate during the unit time exceeds the first threshold number, the number of consecutive times the prediction error exceeds the threshold or the threshold ratio during the unit time exceeds the second threshold number, the ratio of the time when the prediction error exceeds the threshold or the threshold ratio relative to the unit time exceeds the first threshold ratio, or a combination thereof.

If the conditions for regenerating the prediction model are satisfied, the prediction model is regenerated using the training data, and the previously used prediction model is updated with the regenerated prediction model (S235). Here, regeneration of the prediction model is characterized by using a subsequent data set generated from the biometric information measured up to the current time in addition to the previous data set used to create the prediction model stored in the storage unit.

Here, the data set is characterized as a feature vector value generated from first feature values and second feature values.

FIG. 8 is a diagram for explaining an example of calibrating time delay.

As shown in FIG. 8, there is a time delay between the biometric information at the time when the biometric information is actually measured by the body attachable unit (indicated by a dotted line) and the biometric information at the time when the biometric information is received by the communication terminal (indicated by a solid line). The time delay that occurs in this way can be calibrated using known Kalman filters, ARIMA, etc.

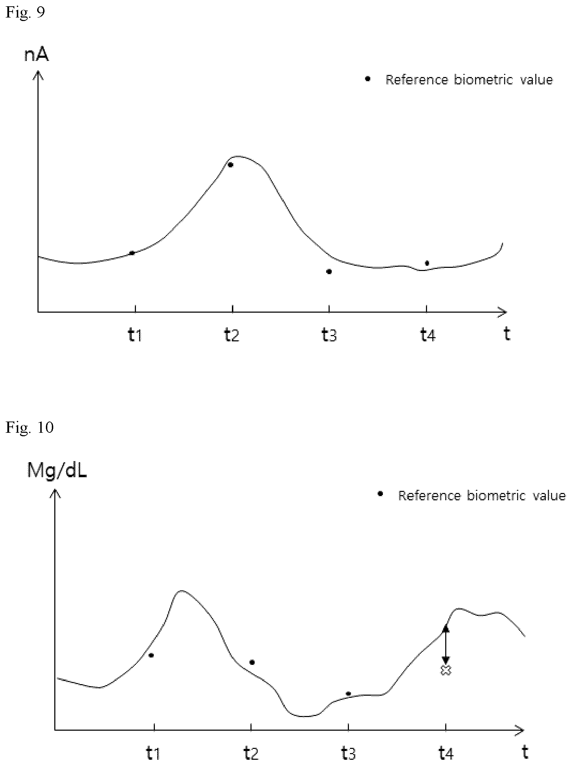

FIG. 9 is a diagram for explaining an example of a method for calibrating unit discrepancy.

As shown in FIG. 9, in a continuous blood glucose measurement system, biometric information received from a body attachable unit should calibrate unit discrepancies using reference biometric values at certain time intervals (t1, t2, t3, t4...), for example, 12 hours, 24 hours, for accuracy.

When calibrating unit discrepancy, a weight can be assigned considering whether the biometric information (indicated by a solid line) is rising or falling, and the calibration can be made by multiplying the calibration slope (A) by the assigned weight. Here, if the biometric information is rising, the weight may be assigned in inverse proportion to the rising speed, and if the biometric information is falling, the weight may be allocated in proportion to the falling speed. For example, if biometric information is rising, the weight may be assigned a low value in inverse proportion to the rising speed (the higher the rising speed, the lower the value, such as 0.90, 0.80, 0.70, etc.), and if biometric information is descending, the weight may be assigned a high value in proportion to the descent speed (the greater the descent speed, the higher the value, such as 1.10, 1.20, 1.30, etc.).

FIG. 10 is a diagram for explaining another example of a method for calibrating unit discrepancy.

As shown in FIG. 10, in the continuous blood glucose measurement system, biometric information received from a body attachable unit should calibrate unit discrepancies using reference biometric values at certain time intervals (t1, t2, t3, t4...), for example, 12 hours, 24 hours, for accuracy.

When calibrating unit discrepancy, it can be calibrated based on the difference between the reference biometric value and the measured biometric value (indicated by a solid line). Based on the difference between reference biometric values and measured biometric values, weights are assigned to reduce the difference (e.g., weights are calculated and assigned so that the calibration slope has the average of reference biometric values and measured biometric values), or only when the difference between the reference biometric value and the measured biometric value is outside the threshold range, a weight can be assigned to reduce the difference.

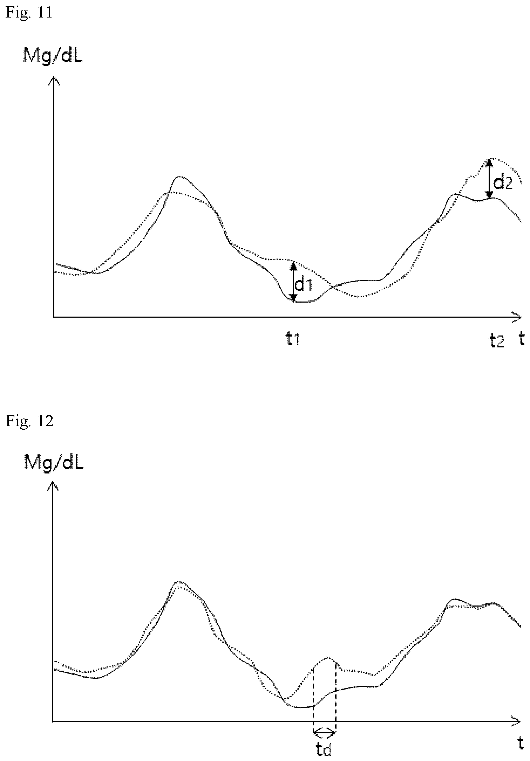

FIGS. 11 and 12 are diagrams for explaining an example of determining whether to relearn a prediction model.

As shown in FIG. 11, when the prediction error (d1) between the predicted biometric value predicted at the first prediction time point (t1) and the measured biometric value actually measured at the first prediction time point is outside the threshold or threshold range, or when the prediction error (d2) between the predicted biometric value predicted at the second prediction time point (t2) and the measured biometric value actually measured at the second prediction time point is outside the threshold or threshold range, the prediction model can be relearned at the first prediction time and the second prediction time, respectively.

Meanwhile, as shown in FIG. 12, if the average of prediction errors between the predicted biometric value predicted for a certain time (td) and the measured biometric value actually measured at the corresponding time exceeds the average of thresholds, the prediction model can be relearned.

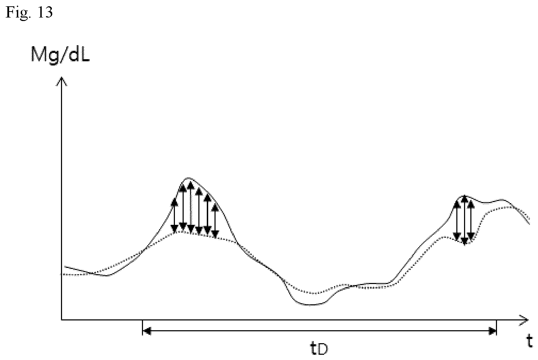

FIG. 13 is a diagram for explaining an example of regenerating a prediction model.

As shown in FIG. 13, when the total number of times the prediction error between the predicted biometric values predicted during a certain unit time (tD) and the measured biometric values actually measured at the corresponding time deviates from the threshold exceeds the threshold number, or when the number of consecutive times the prediction error between the predicted biometric value predicted during a certain unit of time (tD) and the measured biometric value actually measured at the corresponding time deviates from the threshold exceeds the threshold number, or satisfies all combinations thereof, the prediction model can be regenerated.

Meanwhile, the above-described embodiments of the present disclosure can be written as a program that can be executed on a computer, and can be implemented in a general-purpose digital computer that operates the program using a computer-readable recording media.

The computer-readable recording media include storage media such as magnetic storage media (e.g., ROM, floppy disk, hard disk, etc.), optical reading media (e.g., CD-ROM, DVD, etc.), and carrier wave (e.g., transmission via Internet).

The present disclosure has been described with reference to the embodiments shown in the drawings, but these are merely exemplary, and those skilled in the art will understand that various modifications and other equivalent embodiments are possible therefrom. Therefore, the true scope of technical protection of the present disclosure should be determined by the technical concepts of the attached registration claims.

## Claims

1. A method of predicting a biometric value of a user using biometric information measured from a sensor, the method comprising:

   extracting a first feature value from the measured biometric information of the user;
   calibrating the measured biometric information of the user and extracting a second feature value from the calibrated biometric information;
   generating a feature vector value by reducing and combining the first feature value and the second feature value; and
   predicting the biometric value of the user by applying the generated feature vector value to a prediction model.

2. The method of predicting the biometric value according to claim 1,
   wherein the sensor is a sensor partially inserted into body of the user for a certain period of time and continuously measuring the biometric information of the user.

3. The method of predicting the biometric value according to claim 2,

   further includes pre-processing the measured biometric information by removing noise from the measured biometric information,
   wherein the first feature value and the second feature value are extracted from the pre-processed biometric information.

4. The method of predicting the biometric value according to claim 3, wherein:

   the first feature value is directly extracted from the pre-processed biometric information, and
   the second feature value is extracted from the calibrated biometric information generated by calibrating the pre-processed biometric information with respect to time delay and unit discrepancy.

5. The method of predicting the biometric value according to claim 4,
   wherein the unit discrepancy is calibrated based on the pre-processed biometric information or a reference biometric value.

6. The method of predicting the biometric value according to claim 5,
   wherein the unit discrepancy is calibrated by assigning a weight when the pre-processed biometric information increases or decreases.

7. The method of predicting the biometric value according to claim 5,
   wherein the unit discrepancy is calibrated by a weight assigned according to difference between the biometric value determined from the measured biometric information and the reference biometric value.

8. The method of predicting the biometric value according to claim 4, further comprising:

   calculating a prediction error from a difference between a predicted biometric value at a first prediction time and a biometric value actually measured at the first prediction time; and
   determining whether to re-learn the prediction model based on the prediction error.

9. The method of predicting the biometric value according to claim 8,
   wherein if the prediction error is greater than a threshold or a threshold ratio, it is determined that the prediction model is to be re-learned.

10. The method of predicting the biometric value according to claim 8, further comprising
    determining whether to re-generate the prediction model based on expression characteristics of the prediction error during a unit time.

11. The method of predicting the biometric value according to claim 10,
    wherein the expression characteristics are at least one of a number of consecutive times of excess of the prediction error over the threshold or the threshold ratio during the unit time and a total number of times of excess of the prediction error over the threshold or the threshold ratio during the unit time.

12. The method of predicting the biometric value according to claim 8,
    wherein the re-learning of the prediction model or re-generating of the prediction model uses a subsequent data set generated from biometric information of the user measured up to current time except a previous data set which was used to create the prediction model.

Fig. 1

Fig. 2

Fig. 3

**151**

PREPROCESSOR
UNIT

**153**

FIRST FEATURE
VALUE
EXTRACTION UNIT

**155**

CALIBRATION
UNIT

**157**

SECOND FEATURE
VALUE
EXTRACTION UNIT

**159**

FEATURE VECTOR
VALUE
GENERATOR UNIT

Fig. 4

```
         191                        193                      197
          |                         |                        |
┌──────────────────┐      ┌──────────────────┐     ┌──────────────────┐
│                  │      │    CONDITION     │     │                  │
│  PREDICTION ERROR│─────>│  DETERMINATION   │────>│   RELEARNING     │
│  CALCULATION UNIT│      │      UNIT        │     │      UNIT         │
│                  │      │                  │     │                  │
└──────────────────┘      └──────────────────┘     └──────────────────┘
         ▲                         ▲
         │         195             │                         199
         │          |              │                          |
         ▼                         │                ┌──────────────────┐
┌──────────────────┐               │                │                  │
│  PREDICTION ERROR│               │                │   GENERATOR      │
│  CHARACTERISTIC  │<──────────────┘                │     UNIT         │
│ DETERMINATION UNIT│                               │                  │
└──────────────────┘                               └──────────────────┘
```

Fig. 5

START

110
Receiving biometric information

130
Preprocessing biometric information

150
Calibrating biometric information

140
Extracting a first feature value

160
Extracting a second feature value

170
Generating a feature vector value

190
Predicting a biometric value

END

Fig. 6

Fig. 7

```
              ┌─────────┐
              │  START  │
              └────┬────┘
                   │                              231
        ┌──────────▼──────────────────┐        ⌇
        │   Determining  an expression │
        │ characteristics of the prediction error │
        └──────────┬──────────────────┘
                   │
                   │                              233
              ┌────▼─────────────┐              ⌇
              ╱ Regeneration conditions ╲
   NO ◄──────╱  of the prediction model  ╲
             ╲        satisfied?         ╱
              ╲────────┬────────────────╱
                   │  YES                          235
        ┌──────────▼──────────────────┐        ⌇
        │  Regenerating  a prediction  model │
        └──────────┬──────────────────┘
                   │
              ┌────▼────┐
              │   END   │
              └─────────┘
```

Fig. 8

Fig. 9

Fig. 10

Fig. 11

Fig. 12

Fig. 13

## INTERNATIONAL SEARCH REPORT

| International application No. |
| --- |
| **PCT/KR2022/007876** |

### A. CLASSIFICATION OF SUBJECT MATTER

**A61B 5/145**(2006.01)i; **A61B 5/155**(2006.01)i; **A61B 5/00**(2006.01)i; **G16H 50/20**(2018.01)i

According to International Patent Classification (IPC) or to both national classification and IPC

### B. FIELDS SEARCHED

Minimum documentation searched (classification system followed by classification symbols)

A61B 5/145(2006.01); A61B 5/00(2006.01); A61B 5/0488(2006.01); A61B 5/0492(2006.01); A61B 5/24(2021.01); G06N 20/00(2019.01); G06N 20/20(2019.01); H04B 1/40(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 생체값(biometrics), 혈당(blood glucose), 센서(sensor), 예측(predict), 모델(model), 보정(correct), 학습(learning), 변환(convert)

### C. DOCUMENTS CONSIDERED TO BE RELEVANT

| Category* | Citation of document, with indication, where appropriate, of the relevant passages | Relevant to claim No. |
| --- | --- | --- |
| Y | KR 10-2021-0015045 A (SEOUL NATIONAL UNIVERSITY R&DB FOUNDATION et al.) 10 February 2021 (2021-02-10)<br>See claims 1, 2 and 9; and paragraphs [0025], [0039] and [0046]. | 1-12 |
| Y | KR 10-2012-0080270 A (TONGMYONG UNIVERSITY INDUSTRIAL-ACADEMIC COOPERATION FOUNDATION) 17 July 2012 (2012-07-17)<br>See paragraphs [0069]-[0071]. | 1-12 |
| Y | KR 10-2005-0037903 A (KJ HEALTH CARE CO., LTD.) 25 April 2005 (2005-04-25)<br>See claim 1. | 5-7 |
| Y | KR 10-2021-0012791 A (ELECTRONICS AND TELECOMMUNICATIONS RESEARCH INSTITUTE) 03 February 2021 (2021-02-03)<br>See claims 1, 3 and 8; paragraph [0038]; and figures 1 and 3. | 8-12 |
| A | KR 10-2021-0107428 A (ATSENS CO., LTD.) 01 September 2021 (2021-09-01)<br>See entire document. | 1-12 |

☐ Further documents are listed in the continuation of Box C.  ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | "X" document of particular relevance; the claimed invention cannot be considered novel or cannot be considered to involve an inventive step when the document is taken alone |
| "D" document cited by the applicant in the international application | |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
| --- | --- |
| **08 March 2023** | **08 March 2023** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**
**Information on patent family members**

International application No.

**PCT/KR2022/007876**

| Patent document cited in search report | | | Publication date (day/month/year) | Patent family member(s) | | | Publication date (day/month/year) |
|---|---|---|---|---|---|---|---|
| KR | 10-2021-0015045 | A | 10 February 2021 | KR | 10-2383512 | B1 | 06 April 2022 |
| KR | 10-2012-0080270 | A | 17 July 2012 | KR | 10-1208719 | B1 | 06 December 2012 |
| KR | 10-2005-0037903 | A | 25 April 2005 | WO | 2005-039065 | A1 | 28 April 2005 |
| KR | 10-2021-0012791 | A | 03 February 2021 | KR | 10-2434460 | B1 | 22 August 2022 |
| KR | 10-2021-0107428 | A | 01 September 2021 | KR | 10-2427287 | B1 | 01 August 2022 |

Form PCT/ISA/210 (patent family annex) (July 2019)